# FASCICULE DE BREVET EUROPEEN

(11) **EP 1 997 374 B1**
(45) Date de publication et mention de la délivrance du brevet: **02.11.2016**
(21) Numéro de dépôt: 08290484.8
(22) Date de dépôt: 27.05.2008
(51) Int. Cl.: A01N 1/02

(54) **Procédé de rinçage et de conservation d'un organe en vue de sa transplantation et solution mise en oeuvre dans ce procédé**
Spül- und Konservierungsverfahren eines zu transplantierenden Organs und Lösung, die bei diesem Verfahren verwendet wird
Method for rinsing and preserving an organ with a view to transplanting it and solution used in this method

(30) Priorité: 31.05.2007 FR 0703883
(43) Date de publication de la demande: 03.12.2008
(73) Titulaire: MACO PHARMA, 59420 Mouvaux (FR); CHU de Poitiers, 86021 Poitiers Cedex (FR); Université de Poitiers, 86034 Poitiers Cedex (FR); Assistance Publique-Hôpitaux de Paris (APHP), 75004 Paris (FR); Université Pierre et Marie Curie (Paris 6), 75252 Paris Cedex 05 (FR)
(72) Inventeur: Heron, Antoine, 67520 Marlenheim (FR); Eugene, Michel, 86000 Poitiers (FR); Hauet, Thierry, 86280 Saint Benoit (FR); Barrou, François, 75004 Paris (FR); Cinqualbre, Jacques, 67560 Rosheim (FR)
(74) Mandataire: Herrou, Nathalie

(56) Documents cités:
- WO-A-02/41696
- WO-A1-00/69259
- FR-A- 2 723 818
- DUTHEIL, D. ET AL: "Protective effects of PEG 35 000 Da on Renal Cells" AMERICAN JOURNAL OF TRANSPLANTATION, vol. 6, juillet 2006 (2006-07), pages 1529-1540, XP002472088
- NEUZILLET ET AL: "Effects of the Molecular Weight of Peg Molecules (8, 20 and 35 KDA) on Cell Function and Allograft Survival Prolongation in Pancreatic Islets Transplantation" TRANSPLANTATION PROCEEDINGS, ORLANDO, FL, US, vol. 38, no. 7, septembre 2006 (2006-09), pages 2354-2355, XP005656116 ISSN: 0041-1345
- NEUZILLET Y. ET AL.: "Comparaison in vivo de la préservation 'îlots de Langerhans murine" PROGRES EN UROLOGIE, vol. 16, 26 avril 2006 (2006-04-26), pages 73-77, XP002472178
- HAUET THIERRY ET AL: "Polyethylene glycol reduces the inflammatory injury due to cold ischemia/reperfusion in autotransplanted pig kidneys" KIDNEY INTERNATIONAL, vol. 62, no. 2, août 2002 (2002-08), pages 654-667, XP002472087 ISSN: 0085-2538

## Description

L'invention concerne un procédé pour le conditionnement d'un organe en vue de sa transplantation, ainsi qu'une solution pour la mise en oeuvre de ce procédé et un container stérile comprenant une telle solution.

Elle s'applique dans le cas où un organe tel que le foie, rein, coeur et/ou poumon est prélevé chez un donneur. Au cours du prélèvement, les organes sont habituellement rincer par perfusion *in situ* et *ex situ* avec une solution de perfusion, explantés, et conservés dans une solution de conservation. Avant transplantation dans le receveur, l'organe est éventuellement à nouveau rincé.

Il existe un certain nombre de solutions utilisées dans le cadre de la transplantation. Par exemple, la solution UW (commercialisée sous le nom Viaspan® par Fresenius) dont la spécificité est la présence du colloïde HES, est la référence pour la conservation des organes tels que foie, rein, pancréas et intestin.

Cette solution a une concentration élevée en potassium qui compense la perte en potassium de l'organe conservé à basse température en état d'hypothermie. Cependant, l'excès de potassium doit être éliminé de l'organe avant transplantation afin d'éviter un arrêt cardiaque.

La solution de conservation des organes et tissus, commercialisée par Maco Pharma est une solution normo-potassique contenant du polyéthylène glycol (PEG). Cette solution a été développée pour permettre la conservation des organes. Du fait de sa basse teneur en potassium, il n'est pas nécessaire d'effectuer un nouveau rinçage avant implantation.

Cependant, cette solution n'est pas adaptée au rinçage de l'organe au cours du prélèvement. En effet, il se crée des zones hypoperfusées, néfastes pour la reprise du greffon chez le receveur.

Le procédé selon l'invention propose donc d'améliorer cette solution de conservation d'organes, en l'adaptant au rinçage d'organe. La solution conserve ses propriétés de conservation, notamment en limitant les lésions d'ischémie-reperfusion.

L'invention propose également un procédé de conditionnement d'un organe qui optimise les conditions de rinçage de l'organe, en maintenant des bonnes conditions de conservation.

A cet effet, l'invention propose un procédé pour le conditionnement d'un organe en vue d'une transplantation comprenant les étapes successives de :
- rinçage de l'organe, et
- conservation de l'organe dans une solution normopotassique dont la concentration en potassium est inférieure à 10 mM et comprenant une quantité comprise entre 15 et 20 g/L de polyéthylène glycol d'un poids moléculaire de 20 000 Da, caractérisé en ce que le rinçage est réalisé avec une solution de composition identique à celle utilisée dans l'étape de conservation, ladite solution ayant une viscosité comprise entre 0,8 et 1,4 mm²/s à 20°c.

WO02/41 696 décrit un procédé de conservation dans lequel l'organe est rincé avec la même solution que celle dans laquelle il est conservé. La solution normopotassique proposée contient du PEG (20 000 Da) dans une quantité équivalente à 26 g/L.

La transplantation est le transfert d'un organe d'un individu donneur vers un individu receveur. L'individu donneur est un animal humain ou non. Les organes pouvant être transplantés sont les organes abdominaux tel que le foie, rein, pancréas et colon, mais aussi les autres organes tels que le coeur et le poumon. Les organes sont prélevés notamment sur un cadavre ou un volontaire sain.

Le procédé de l'invention est un procédé *in vitro,* c'est-à-dire qu'il concerne l'organe une fois celui-ci prélevé du donneur.

Le succès de la transplantation dépend entre autre de la qualité de l'organe à transplanter, de son rinçage et de sa conservation afin de limiter les lésions formées lors des phases d'ischémie et reperfusion.

Pour préparer un organe en vue d'une transplantation, l'organe est d'abord rincé avec une solution de rinçage afin d'éliminer le sang résiduel du greffon.

Le rinçage est réalisé par perfusion de l'organe par la solution, selon les conditions de température allant de l'hypothermie à la normothermie. Avantageusement, le rinçage est réalisé avec une solution refroidie à 4°C pour refroidir l'organe.

Ensuite, l'organe est plongé dans une solution de conservation apte à prévenir les lésions provoquées par l'ischémie.

Avantageusement, l'organe est conservé à basse température, notamment à 4°C, pour le maintenir en hypothermie.

Selon l'invention, les solutions de rinçage et la solution de conservation ont une composition identique.

La solution est normopotassique, c'est-à-dire qu'elle comporte une concentration basse d'ions potassium, notamment proche de celle existant dans le plasma.

La solution utilisée dans
l'invention comprend du polyéthylène glycol (PEG) de haut poids moléculaire, c'est-à-dire de 20 000 Da. Cette molécule colloïdale, en exerçant une pression oncotique à l'intérieur des vaisseaux, est particulièrement efficace pour lutter contre l'oedème cellulaire et interstitiel. Ainsi il ne se forme pas de nécroses localisées.

En outre, le PEG diminue l'immunogénicité du greffon, liée aux lésions d'ischémie - reperfusion.

Cependant, le PEG est un composé visqueux qui augmente la viscosité de la solution. Même si des solutions à la viscosité élevée telle que la solution UW sont adaptées au rinçage des organes, la présence d'une trop grande quantité de PEG dans une solution de conservation la rend inadaptée pour cette utilisation.

Selon l'invention, la solution de rinçage et conservation possède une viscosité comprise entre 0,8 et 1,4 mm²/s à 20°C, notamment entre 1 et 1,3 mm²/s à 20°C.

Cette viscosité particulière est suffisamment faible pour que l'organe rincé ne contienne aucune zone hypoperfusée, c'est-à-dire que la décoloration de l'organe due à la perfusion de la solution est homogène. Ces zones hypoperfusées donnent lieu à une nécrose de conservation focalisée, avec relarguage cytolytique.

La solution utiliséedans
l'invention possède une viscosité comprise entre 0,8 et 1,4 mm²/s à 20°C, notamment 1 et 1,3 mm²/s, induite par la présence et les propriétés du polyéthylène glycol à des concentrations données.

Ces caractéristiques confèrent à la solution des propriétés rhéologiques adaptées à une perfusion optimale des organes et notamment du foie. La viscosité reste inférieure à celle du sang et peut donc circuler dans tout le système vasculaire de l'organe à transplanter. La solution garde cependant des bonnes propriétés de conservation.

Pour le PEG 20 000 Da, une quantité de 15 g/L permet d'obtenir une viscosité d'environ 1,05 mm²/s à 20°C.

Cette viscosité particulière est obtenue en utilisant une concentration définie de PEG, suffisante pour garantir ses propriétés anti-oedème et d'immunocammouflage.

Notamment, la solution est utilisée pour le rinçage et la conservation du coeur.

Dans ce cas, la solution utilisée dans
l'invention peut comprendre en outre un agent cardioprotecteur tel que le 2,3-butanedione monoxime.

Lasolution utilisée dans l'invention comprend en outre :
un agent imperméant,
un tampon,
une solution d'électrolytes,
de l'eau ppi.

L'agent imperméant peut être l'acide lactobionique, l'acide gluconique ou un de leurs sels ou un saccharide tel que le raffinose, le sucrose ou le glucose. Il empêche le passage de l'eau au travers les membranes cellulaires et évite ainsi l'oedème cellulaire.

Le tampon est choisi par exemple parmi les tampons physiologiques (sulfate ou carbonate) ou synthétique (HEPES), de sorte à maintenir la solution de perfusion et de conservation à un pH physiologique, compris entre 7 et 8, notamment environ 7,5.

La solution d'électrolytes contient des ions sodium, potassium, calcium et/ou magnésium et/ou d'autres ions.

Avantageusement, la solution utiliséedans l'invention est de type extracellulaire, sa concentration d'ions sodium est élevée, notamment supérieure à 40 mM, notamment supérieure à 115 mM.

La solution est normopotassique, c'est-à-dire que sa concentration en potassium est inférieure à 10 mM. Ainsi, il n'est pas nécessaire de rincer l'organe avant de l'implanter dans le receveur.

Lasolution utiliséedans
l'invention possède une osmolarité physiologique proche de celle du plasma comprise entre 300 et 380 mOsm/kg H₂O, notamment 320 mOsm/Kg H20.

Dans un exemple particulier, la solution de rinçage et conservation comprend du PEG 20 000 Da dans une solution de Krebs dont la composition pour 1 L de solution est la suivante :
0,45 g de chlorure de potassium,
0,24 g de chlorure de magnésium hexahydrate,
6,94 g de chlorure de sodium,
0,17 g de chlorure de calcium dihydrate,
2,2 g de D-glucose monohydrate,
2,1 g de carbonate de sodium,
eau ppi.

Lorsque la solution comprend 20 g/L de PEG 20 000, elle possède une viscosité de 1,19 mm²/s à 20°C. Avec 15 g/L de PEG 20 000, la solution possède une viscosité de 1,05 mm²/s à 20°c.

La solution de rinçage et de conservation est rendue stérile par filtration et conditionnée dans un container tel qu'un flacon ou une poche souple, avantageusement imperméable à l'oxygène.

Le container comprend une embouchure munie d'un bouchon amovible. Une fois le bouchon retiré de l'embouchure la solution utilisée dans
l'invention est déversée facilement et rapidement dans un récipient contenant l'organe à conserver.

Selon une variante, le container comprend un connecteur pour ligne de perfusion. Après connexion d'une ligne de perfusion sur le connecteur du container, la solution contenue dans le container est facilement perfusée dans l'organe à rincer.

Selon un autre variante, le container comprend une embouchure munie d'un bouchon amovible et un connecteur pour ligne de perfusion pour réaliser successivement les opérations de rinçage et conservation avec la même solution.

La solution utilisée dans l'invention est utilisée pour le rinçage et la conservation des organes selon le procédé de l'invention.

Cette solution est adaptée à tous les organes abdominaux. Pour le coeur, la solution comprend en outre 3,03 g de 2,3-butanedione monoxime en tant qu'agent cardioprotecteur.

Elle peut être également utilisée pour la conservation des tissus ou greffons vasculaires tels que les veines, artères, valves, vaisseaux. En combinaison du diméthyl sulfoxide, le glycérol ou toute autre molécule protectrice, la solution peut aussi être utilisée pour la cryoconservation des tissus.

Exemple 1 : Composition et viscosité de différentes solutions de conservation.

Les viscosités de différentes solutions utilisée dans l'invention (Tableau 1) ainsi que des solutions commerciales de Maco Pharma (S7) et UW ont été déterminées à 20°C à l'aide d'un viscosimètre Cannon-Fenske (Schott type 511-01) et sont consignées dans le tableau 2.

**Tableau 1**

| S5-S7 | quantité |
|---|---|
| Chlorure de potassium | 0,45 g |
| Chlorure de magnésium hexahydrate | 0,24 g |
| Chlorure de sodium | 6,94 g |
| Chlorure de calcium dihydrate | 0,17 g |
| D-glucose,H20 | 2,2 g |
| Carbonate de sodium | 2,1 g |
| Polyéthylène glycol | variable |
| Eau ppi | 11 |

**Tableau 2**

| **Solutions** | **Viscosité (mm²/s)** |
|---|---|
| SS - PEG 20 kDa - 1Sg/L | 1,0S |
| S6 - PEG 20 kDa - 20 gL | 1,19 |
| S7 - PEG 20kDa - 30 g/L | 1,SS |
| UW (Viaspan®) | 2,38 |

La solution S7 n'est pas représentative de l'invention.
Exemple 2 : Etude de l'effet protecteur d'une solution de conservation utilisée dans l'invention sur le greffon hépatique.

Dans l'étude qui suit, le foie, du fait de sa sensibilité à l'ischémie et à la géographie de son réseau vasculaire, a été pris comme standard pour tester l'efficacité de rinçage d'une solution utilisée dans l'invention.

Le modèle porcin a été choisi pour mener l'expérimentation animale avant d'envisager toute application clinique chez homme.

Les animaux utilisés pour l'allotransplantation sont des porcelets (Large White) d'environ 30 kg.

Le greffon hépatique est prélevé sur un donneur suivant un protocole chirurgical adapté et placé dans un container remplie de solution S5, S6 ou S7 à 4°C.

Une perfusion *ex-vivo* du greffon par les voies intra-portale et intra-artérielle est alors réalisée avec 500 ml de la solution SS, S6 ou S7.

Le greffon est alors implanté chez l'animal receveur, on observe la recoloration homogène du greffon hépatique, sans aucune mal-façon des anastomoses.

### Résultats

Les critères pour évaluer l'efficacité de la solution dans un modèle de transplantation hépatique chez le porc sont la fonction du greffon et la survie des animaux au 7èmejour post-transplantation.

Les études histologiques sont réalisées par l'observation microscopique des coupes comme suit :
- Foie 1 : foie après 8h de conservation et avant transplantation
- Foie 2 : foie transplanté prélevé après 1 h de revascularisation
- Foie 3 : foie au décès de l'animal

Les résultats sont résumés ci-dessous :
- Solution S7 (9 animaux allo transplantés) : Très mauvais taux de survie des animaux : décès des animaux quelques heures post transplantation, un seul animal survivant à J7.
- Solution S6 (7 animaux allo transplantés) : Taux de survie des animaux moyen : décès quelques heures ou jours post transplantation, 3 animaux survivants à J7, soit 43% de survie avec une bonne fonction du greffon.
- Solution S5 (7 animaux allo transplantés) : Très bon taux de survie des animaux à J7 (86%), un seul décès pour cause chirurgicale avec une bonne fonction du greffon.

En conclusion, l'étude préclinique ne montre pas de différences significatives entre le groupe d'animaux dont le foie a été rincé avec la solution S5 versus la solution UW (8 animaux allo transplantés selon un groupe historique ) en termes de fonction du greffon et de survie des animaux.

Les observations macroscopiques des foies ont révélé un rinçage efficace avec la solution S5 et une recoloration homogène au cours de la revascularisation.

Les examens anatomo-pathologiques,
- après 8h de conservation hypothermique montrent un tissu hépatique normal,
- après 1 h de revascularisation post transplantation, ne montrent pas de congestion, pas d'infiltration, pas de lésions de l'endothélium, pas de nécrose hépatocytaire.
- à J7 après euthanasie de l'animal, ne montrent pas d'infiltration de la structure portale, pas de nécrose, pas de congestion. Dans deux cas, il a été observé un rejet cellulaire modéré.

Cette étude montre la possibilité d'effectuer un rinçage rapide avec absence d'hématies évacuée pendant une conservation de 24 h dans la solution S5. La reperfusion est rapide et homogène.

### Exemple 3 : Conservation du rein

L'animal choisi est le porc Large White. Pour des raisons pratiques, des mâles dont le poids varie entre 35 et 50 kilogrammes (INRA, Domaine du Magneraud, Surgères, France) ont été utilisés.

Le rein prélevé est plongé dans du sérum physiologique à 4°C. Un cathéter (14 gauges) est introduit dans l'artère rénale, permettant le lavage du rein par 300 à 500 ml de solution S5 ou S6. Après pesée, le rein est placé dans un récipient stérile contenant la solution de conservation S5 ou S6, et conservé à 4°C.

Les animaux donneurs sont soit transplantés après 30 minutes d'ischémie froide soit isolés en cage et transplantés après 24 h d'ischémie froide avec leur propre rein.

### Résultats des études histomorpholoaiaues durant la conservation

Les prélèvements ont été réalisés à partir des reins conservés, après lavage avec les solutions étudiées.

L'évolution des lésions a été déterminée selon une classification concernant essentiellement le Détachement des cellules Endothéliales dans la Lumière tubulaire (DEL) et la Perte de la Bordure en Brosse (PBB).

Cette classification a été établie selon 5 classes de 1 a 5.
1 : pas de lésion
2 : <10%
3 : entre 11 et 25%
4 : entre 26 et 50%
5 : >50%

Les prélèvements ont été faits sur 6 animaux (3 pour le groupe traité avec S5 et 3 pour le groupe traité avec S6) au temps 0, puis à 1 h, 6h, 20h, 24h et 48h de conservation.

**Les résultats de la graduation des lésions sont résumés dans le tableau 3.**

| | PEG 15 g/l (S5) | | | | | | PEG 20 g/1 (S6) | | | | | |
|---|---|---|---|---|---|---|---|---|---|---|---|---|
| | DEL | | | PBB | | | DEL | | | PBB | | |
| Animal | N°1 | N°2 | N°3 | N°1 | N°2 | N°3 | N°1 | N°2 | N°3 | N°1 | N°2 | N°3 |
| Oh | 1 | 1 | 1 | 1 | 1 | 1 | 1 | 1 | 1 | 1 | 1 | 1 |
| 1h | 1 | 1 | 1 | 1 | 1 | 1 | 1 | 1 | 1 | 1 | 1 | 1 |
| 6h | 1 | 1 | 1 | 1 | 1 | 1 | 1 | 2 | 1 | 1 | 2 | 1 |
| 20h | 2 | 2 | 2 | 2 | 2 | 2 | 2 | 2 | 2 | 2 | 2 | 2 |
| 24 | 2 | 2 | 2 | 2 | 2 | 2 | 2 | 3 | 2 | 2 | 3 | 2 |
| 48h | 3 | 4 | 3 | 3 | 4 | 4 | 4 | 4 | 4 | 4 | 4 | 4 |

### Résultats des études fonctionnelles durant la première semaine après autotransplantation.

Deux groupes de 4 animaux ont été transplantés avec leur propre rein perfusé et conservé dans les solutions S5 et S6, respectivement.

Les résultats montrent que les solutions S5 et S6 ne sont pas délétères pour l'organe et que la diminution de concentration de PEG 20000 à 15g/L a une influence favorable avec une production d'urine plus précoce et une rediminution de la créatininémie plus rapide.

Les qualités de rinçage et de conservation d'une solution utilisée dans l'invention sont ainsi démontrées.

## Revendications

1. Procédé pour le conditionnement d'un organe en vue d'une transplantation comprenant les étapes successives de :
rinçage de l'organe, et
conservation de l'organe dans une solution normopotassique dont la concentration en potassium est inférieure à 10 mM et comprenant une quantité comprise entre 15 et 20 g/L de polyéthylène glycol d'un poids
moléculaire de 20 000 Da,
**caractérisé en ce que** le rinçage est réalisé avec une solution de composition identique à celle utilisée dans l'étape de conservation, ladite solution ayant une viscosité comprise entre 0,8 et 1,4 mm²/s à 20°c.

2. Procédé selon la revendication 1, **caractérisé en ce que** le rinçage de l'organe est réalisé par perfusion de la solution.

3. Procédé selon l'une quelconque des revendications 1 à 2, **caractérisé en ce que** l'organe est choisi parmi les organes abdominaux.

4. Procédé selon l'une quelconque des revendications 1 à 2, **caractérisé en ce que** l'organe est le coeur et la solution comprend en outre un cardioprotecteur.

5. Procédé selon l'une quelconque des revendications 1 à 4, **caractérisé en ce que** la solution possède une viscosité comprise entre 1 et 1,3 mm²/s.

6. Procédé selon l'une quelconque des revendications 1 à 5, **caractérisé en ce que** la solution comprend en outre :
un agent imperméant,
un tampon,
une solution d'électrolytes
de l'eau ppi.

7. Procédé selon l'une quelconque des revendications 1 à 6, **caractérisé en ce que** la solution possède une osmolarité comprise entre 300 et 380 müsm/kg H20.

8. Procédé selon l'une quelconque des revendications 1 à 7, caractérisé en ce la solution comprend dans 1 L d'eau ppi :
0,45 g de chlorure de potassium,
0,24 g de chlorure de magnésium hexahydrate,
6,94 g de chlorure de sodium,
0,17 g de chlorure de calcium dihydrate,
2,2 g de D-glucose monohydrate,
2,1 g de carbonate de sodium,
15 g de PEG.

## Patentansprüche

1. Verfahren zur Konditionierung eines zu transplantierenden Organs, das die folgenden aufeinander folgenden Schritte umfasst:
- Spülen des Organs, und
- Konservieren des Organs in einer Normalkaliumlösung, deren Kaliumkonzentration kleiner 10 mM ist und eine Menge im Bereich zwischen 15 und 20 g/L Polyethylenglycol mit einem Molekulargewicht von 20 000 Da umfasst,
**dadurch gekennzeichnet, dass** das Spülen mit einer Lösung durchgeführt wird, deren Zusammensetzung mit der identisch ist, die im Konservierungsschritt verwendet wird, wobei die Lösung bei 20 °C eine Viskosität im Bereich zwischen 0,8 und 1,4 mm²/s aufweist.

2. Verfahren nach Anspruch 1, **dadurch gekennzeichnet, dass** das Spülen des Organs durch Perfusion der Lösung durchgeführt wird.

3. Verfahren nach einem der Ansprüche 1 bis 2, **dadurch gekennzeichnet, dass** das Organ aus den Bauchorganen ausgewählt ist.

4. Verfahren nach einem der Ansprüche 1 bis 2, **dadurch gekennzeichnet, dass** das Organ das Herz ist und die Lösung ferner einen Kardioprotektor umfasst.

5. Verfahren nach einem der Ansprüche 1 bis 4, **dadurch gekennzeichnet, dass** die Lösung eine Viskosität im Bereich zwischen 1 und 1,3 mm²/s aufweist.

6. Verfahren nach einem der Ansprüche 1 bis 5, **dadurch gekennzeichnet, dass** die Lösung außerdem Folgendes umfasst:
- einen Impermeanten,
- einen Puffer,
- eine Elektrolytlösung
- Wasser für Injektionszwecke.

7. Verfahren nach einem der Ansprüche 1 bis 6, **dadurch gekennzeichnet, dass** die Lösung eine Osmolarität im Bereich zwischen 300 und 380 mOsm/kg H2O aufweist.

8. Verfahren nach einem der Ansprüche 1 bis 7, **dadurch gekennzeichnet, dass** die Lösung in 1 l Wasser für Injektionszwecke Folgendes umfasst:
- 0,45 g Kaliumchlorid,
- 0,24 g Magnesiumchlorid-Hexahydrat,
- 6,94 g Natriumchlorid,
- 0,17 g Calciumchlorid-Dihydrat,
- 2,2 g D-Glucose-Monohydrat,
- 2,1 g Natriumcarbonat,
- 15 g PEG.

## Claims

1. A method for conditioning an organ with a view to transplantation, comprising the following steps in succession:
- flushing the organ, and
- preserving the organ in a normal-potassium solution which has a potassium concentration of less than 10 mM and comprises a quantity in the range 15 to 20 g/L of polyethylene glycol with a molecular weight of 20 000 Da,
**characterized in that** the flushing is carried out with a solution with a composition which is identical to that used in the preservation step, said solution having a viscosity in the range 0.8 to 1.4 mm2/s at 20°C.

2. The method according to claim 1, **characterized in that** the organ is flushed by perfusion of the solution.

3. The method according to claim 1 or 2, **characterized in that** the organ is selected from the abdominal organs.

4. The method according to claim 1 or 2, **characterized in that** the organ is the heart and the solution further comprises a cardioprotector.

5. The method according to any one of claims 1 to 4, **characterized in that** the solution has a viscosity in the range 1 to 1.3 mm²/s.

6. The method according to any one of claims 1 to 5, **characterized in that** the solution further comprises:
- an impermeant,
- a buffer,
- a solution of electrolytes,
- WFI water.

7. The method according to any one of claims 1 to 6, **characterized in that** the solution has an osmolarity in the range 300 to 380 mOsm/kg H2O.

8. The method according to any one of claims 1 to 7, **characterized in that** the solution comprises, in 1 L of WFI water:
- 0.45 g of potassium chloride,
- 0.24 g of magnesium chloride hexahydrate,
- 6.94 g of sodium chloride,
- 0.17 g of calcium chloride dihydrate,
- 2.2 g of D-glucose monohydrate,
- 2.1 g of sodium carbonate,
- 15 g of PEG.
